# EUROPÄISCHE PATENTSCHRIFT

(11) **EP 0 965 338 B1**
(45) Veröffentlichungstag und Bekanntmachung des Hinweises auf die Patenterteilung: **21.01.2004**
(21) Anmeldenummer: 99103356.4
(22) Anmeldetag: 20.02.1999
(51) Int. Cl.: A61K 31/025, A61P 27/02

(54) **Sauerstoffarme Perfluoroalkane für die Ophthalmologie**
Low oxygen content perfluoroalkanes for ophthalmologic use
Perfluoroalkanes pauvres en oxygène pour usage ophthalmologique

(30) Priorität: 18.03.1998 DE 19811683
(43) Veröffentlichungstag der Anmeldung: 22.12.1999
(73) Patentinhaber: BAUSCH & LOMB INCORPORATED, Rochester, New York 14604-2701 (US)
(72) Erfinder: Kobuch, Karin, Dr., 93080 Pentling (DE); Gabel, Veit-Peter, Prof., 93049 Regensburg (DE); Dresp, Joachim, Dr., 81825 Munchen (DE); Menz, Dirk-Henning, Dr., 86240 Diedorf (DE)
(74) Vertreter: Maiwald Patentanwalts GmbH

(56) Entgegenhaltungen:
- US-A- 4 490 351
- US-A- 5 397 805
- WILSON C A ET AL: "Perfluorinated organic liquid as an intraocular oxygen reservoir for the ischemic retina." INVESTIGATIVE OPHTHALMOLOGY AND VISUAL SCIENCE, (1995 JAN) 36 (1) 131-41., XP002115430
- CRINGLE, S. J. ET AL: "Intravitreal perfluorocarbon and oxygen delivery in induced retinal ischaemia." HOGAN, M. C. [EDITOR];MATHIEU-COSTELLO, O. [EDITOR]; POOLE, D. C. [EDITOR]; WAGNER, P. D. [EDITOR]. ADVANCES IN EXPERIMENTAL MEDICINE AND BIOLOGY, (1994) VOL. 361, PP. 303-311. ADVANCES IN EXPERIMENTAL MEDICINE AND BIOLOGY; OXYGEN TRANSPORT TO TISSUE, XP002115431
- BRAUN R D ET AL: "New perfluorocarbon emulsion improves tissue oxygenation in cat retina." JOURNAL OF APPLIED PHYSIOLOGY, (1992 MAY) 72 (5) 1960-8., XP002115432
- THORESON W B ET AL: "Effects of using the oxygen -carrying fluorocarbon, FC43, on the ERG of the arterially perfused cat eye." CURRENT EYE RESEARCH, (1989 MAY) 8 (5) 487-98., XP002115433
- AUGUSTIN A J ET AL: "Effects of perfluorooctylbromide and vitamin E on ischemia induced retinal oxidative tissue damage." EXPERIMENTAL EYE RESEARCH, (1998 JAN) 66 (1) 19-24., XP002115434

## Beschreibung

Die Erfindung betrifft die Verwendung eines Fluorcarbons mit einem Gehalt an gelöstem Sauerstoff unter 6 vol. % zur Herstellung eines Behandlungsmittel für die Ophthalmologie.

Unter dem Begriff "Fluorcarbon" im Sinne dieser Beschreibung sind dabei entweder Verbindungen aus der Klasse der gesättigten, perfluorierten Kohlenwasserstoffe zu verstehen, bei denen sämtliche Wasserstoffatome durch Fluoratome ersetzt wurden, oder partiell fluorierte Alkane mit der Summenformel R_{F}R_{H} bzw. R_{F}R_{H}R_{F}, die im flüssigen oder gelförmigen Zustand vorliegen können. Hierbei versteht man unter R_{F} einen Perfluoralkylrest und unter R_{H} einen Alkylrest. Derartige Substanzen sowie ihre Verwendungsmöglichkeiten in Medizin und Technik sind beispielsweise in den Druckschriften DE 42 05 341 A1, US 5,275,669, US 4,490,351, EP 493 677 A2 und DE 195 36 504 A1 beschrieben.

Diese Fluorcarbone sind praktisch immer hochgradig mit Sauerstoff gesättigt, wobei der Sauerstoff in physikalischer Lösung vorliegt. Bei Erhöhung des Sauerstoffpartialdruckes können Fluorcarbone bis zu 50 Vol.-% Sauerstoff speichern. Diese verbindungstypische Eigenschaft war Ausgangspunkt für die Erschließung vielfältiger technischer Anwendungen dieser Verbindungen (vgl. den Artikel von B. Cornils "Fluorous biphasic systems" in Angew. Chemie 1997, 109, 2147) sowie medizinischer Anwendungen, z.B. als Blutersatzstoffe, als Mittel für die Flüssigbeatmung, als Sauerstoffträger in Salben usw. Fluorcarbone mit einem geringen Anteil an gelöstem Sauerstoff nehmen in Kontakt mit Luft spontan und sehr schnell so lange Sauerstoff auf und lösen diesen, bis sich ein Gleichgewicht zwischen gelöstem Sauerstoff und Sauerstoffgehalt der umgebenden Atmosphäre eingestellt hat. Beschrieben wurden diese medizinischen Anwendungen der Fluorcarbone als Sauerstoffträger beispielsweise in den oben genannten Druckschriften und in der Arbeit von K. C. Lowe "Properties and Biomedical Applications of PFC and their emulsions", in Org. Fluorine Chem.: Principles and Commercial Appl., Plenum Press, NY 1994. Darüberhinaus wurde die Anwendung von Fluorcarbonen auch als Behandlungsmittel in der Ophthalmologie wiederholt beschrieben, beispielsweise in der europäischen Patentanmeldung 563 446 A1. Insbesondere dienen die Fluorcarbone als Netzhauttamponade bei der Behandlung von Netzhautablösungen, wobei ihre Dichte und ihre Oberflächeneigenschaften von besonderer Bedeutung sind.

Um über eine intraoperative Behandlung hinaus als ophtalmologisches Behandlungsmittel verwendbar zu sein, müssen die Fluorcarbone über einen längeren Zeitraum anstelle des zuvor entfernten Glaskörpers in der Augenhöhle belassen werden, um z.B. einen zeitlich andauernden, beständigen Druck auf die Netzhaut auszuüben.

Es hat sich allerdings gezeigt, daß die bekannten Fluorcarbone nur über einen beschränkten Zeitraum im Auge belassen werden können, vgl. US 5,037,384. Bereits nach kurzer Verweildauer im Auge treten nämlich Schädigungen der Netzhaut und des Gefäßsystems auf, deren Ursprung weitgehend unbekannt ist und unter anderem auf die hohe Dichte der Verbindungen zurückgeführt wird. Aufgrund der starken Bindungsenergie zwischen Kohlenstoff und Fluor sind die Fluorcarbone nämlich chemisch inert und werden daher nicht durch metabolische Reaktionen abgebaut.

Es besteht daher die Aufgabe, ein Behandlungsmittel für die Ophthalmologie so auszubilden, daß auch bei längerer Anwendungsdauer keine oder nur geringe Schädigungen der Netzhaut und der angrenzenden intraokulären Strukturen auftreten.

Gelöst wird diese Aufgabe durch die Verwendung eines Fluorcarbons mit einem Gehalt an gelöstem Sauerstoff unter 6 Vol.-%. Vorteilhafte Ausgestaltungen sind den Unteransprüchen entnehmbar.

Der in den Fluorcarbonen üblicherweise gelösten Sauerstoffmenge wurde bei der Schädigung der Netzhaut und der angrenzenden intraokulären Strukturen bisher keine Bedeutung beigemessen. Soweit dieses Thema in der Literatur überhaupt angesprochen wurde, beispielsweise in der Arbeit von A.J. Augustin, M. Spitznas, F.H.J. Koch, T. Böker, J. Lutz in Graefes Arch. Clin. Exp. Ophthalm. 1995, 233, 45 - 47 Local Effects of Different Perfluorochemical Agents", war die Schlußfolgerung, daß die im Tierversuch beobachteten Schäden der Netzhaut und des intraokulären Systems von anderen Faktoren hervorgerufen werden müssen, beispielsweise von den in den Emulsionen enthaltenen Tensiden. Dies wurde in der genannten Arbeit dadurch bestärkt, daß bei Verwendung von reinen (aber Sauerstoff enthaltenden) Fluorcarbonen toxische Effekte nicht beobachtet werden konnten.

Auch in anderen Arbeiten wurden die durch Fluorcarbone hervorgerufenen Schädigungen immer auf andere Eigenschaften dieser Substanzen und nicht auf deren Lösevermögen für Sauerstoff zurückgeführt. Im Gegenteil wurde das Sauerstofflösevermögen dieser Substanzen wiederholt als Vorteil für ophthalmologische Anwendungen beschrieben.

Die Erfinder erkannten, daß die teilweise beobachtete toxische Wirkung bei Langzeitanwendungen zu einem erheblichen Anteil auf den pysikalisch gelösten Sauerstoff (bei gleichzeitig geringen Anteilen an gelöstem Kohlendioxid) in den Fluorcarbonen zurückzuführen ist. Diese, im Widerspruch zur derzeitigen Lehrmeinung stehende Erkenntnis führt zu der erfindungsgemäßen Reduzierung des Sauerstoffgehalts der Fluorcarbone bei ihrer Verwendung als Behandlungsmittel in der Ophthalmologie.

Untersuchungen haben gezeigt, daß Fluorcarbone, deren Sauerstoffgehalte nicht gezielt reduziert wurden, nach Einbringung in den Glaskörperraum eines Kaninchenauges zu einer Schädigung der retinalen Blutgefäße führten. In der Fluoreszenzangiographie zeigt sich hierbei eine Verengung der Gefäße ab dem zweiten postoperativen Tag. Im weiteren zeitlichen Verlauf zeigen sich Gefäßverschlüsse, eine Rarefizierung des Kapillarbettes und die Ausbildung von Mikroaneurysmen. Histologisch zeigen flache Präparationen der retinalen Blutgefäße Kaliberschwankungen im Bereich der Gefäßwände und einen Verlust an Perizyten und Endothelzellen aus den Gefäßwänden. Kontrollversuche mit erfindungsgemäß desoxygenierten Fluorcarbonen zeigten hingegen normale retinale Blutgefäße.

Ein Ausführungsbeispiel der Erfindung wird im Folgenden näher beschrieben, wobei zunächst einige Verfahren zur Herstellung der für die erfindungsgemäße Verwendung vorgesehenen Fluorcarbone beschrieben werden.

Fluorcarbone weisen unter Normalbedingungen und unter reiner Sauerstoffatmosphäre einen hohen Anteil physikalisch gelösten Sauerstoffs auf. Dieser beträgt bis zu 50 Vol.-%. Unter Raumluft liegt der Sauerstoffanteil noch immer bei ca. 8 Vol.-%. Zur Erzeugung der für die erfindungsgemäße Verwendung notwendigen sauerstoffarmen Fluorcarbone müssen die unter Normalbedingungen vorliegenden Fluorcarbone zunächst desoxygeniert werden. Anschließend ist sicherzustellen, daß der geringe, bei der Desoxygenierung erzielte Sauerstoffgehalt bis zur Verwendung des Fluorcarbons erhalten bleibt.

Als geeignete Verfahren zur Desoxygenierung können bekannte Spülvorgänge mit anderen Gasen als Sauerstoff eingesetzt werden, wobei die Spülung durch Temperierung und/oder aufeinanderfolgendes Evakuieren und Gasspülen verbessert werden kann.

Besonders effektiv sind Verfahren zur Desoxygenierung unter Verwendung von Sauerstoffgettern in heterogener Phase. Als heterogene Sauerstoffgetter sind niedervalente Metalloxide, insbesondere reduziertes Cu₂O besonders geeignet, die bisher hauptsächlich zur Sauerstoffabtrennung in Gasen genutzt werden. Dabei können die heterogenen Sauerstoffgetter als Füllung von Absorberpatronen eingesetzt werden, durch die die Fluorcarbone geleitet werden. Kombiniert man diese Absorberpatronen mit Sterilfiltern, so kann das Fluorcarbon unmittelbar vor seinem Einsatz als ophthalmologisches Behandlungsmittel desoxygeniert werden.

Aufgrund des sehr starken Lösevermögens der Fluorcarbone für Sauerstoff ist dafür zu sorgen, daß die einmal desoxygenierten Fluorcarbone bis zu ihrer Verwendung in sauerstoffarmen Zustand bleiben, was in der Regel nur durch abdichtendes Einschweißen gelingt.

Um sicherzustellen, daß auch Fluorcarbone, welche anderweitig, beispielsweise in Flaschen, verpackt sind, bis zu ihrer Verwendung sauerstoffarm bleiben, können die genannten heterogenen Sauerstoffgetter auch den Fluorcarbonen zugesetzt werden. In diesem Fall sind die Sauerstoffgetter allerdings unmittelbar vor dem Einsatz der Fluorcarbone als Behandlungsmittel in der Ophthalmologie abzutrennen, was beispielsweise durch Filtration erfolgen kann.

Alternativ dazu können die heterogenen Sauerstoffgetter im überstehenden Gasraum plaziert werden, wobei sich ihre den desoxygenierten Zustand erhaltende Wirkung über Diffusionsvorgänge via Gasphase entfaltet. Ein weiterer Vorteil der Verwendung von heterogenen Sauerstoffgettern besteht darin, den Partialdruck anderer gelöster Gase in vorzüglicher Weise einstellen zu können, was bei Desoxygenierungen mittels durchströmender Gase nicht ohne besondere zusätzliche Verfahren gelingt. Die Einstellung des Gehaltes anderer physikalisch gelöster Gase wie Stickstoff oder Kohlendioxid oder Stickstoffoxid kann somit leicht auf die für die jeweiligen Anwendungen vorteilhaftesten Werte erfolgen.

Wünscht man nach vollständiger Desoxygenierung der Fluorcarbone eine bestimmte, definierte Sauerstoffkonzentration, so kann diese durch anteiliges Mischen mit der gleichen, jedoch an Luft gelagerten und daher mit Sauerstoff angereicherten Substanz erfolgen, da die Gleichgewichtskonzentration des Sauerstoffs in dem unter Atmosphärenbedingungen gelagerten Fluorcarbon ein sehr stabiler und reproduzierbarer Wert ist und bei 8 Vol.-% liegt.

Ein von null verschiedener Wert des Sauerstoffgehalts des Fluorcarbons empfiehlt sich beispielsweise bei einer ischämischen Netzhaut, der bewußt Sauerstoff zuzuführen ist, jedoch nicht so viel, daß eine toxische Schädigung der Netzhaut eintritt.

Ferner kann das sauerstoffarme Fluorcarbon auch mit anderen Gasen versetzt werden, beispielsweise mit Stickstoff, Kohlendioxid oder Stickstoffoxid (NO). Weil in zerebralen Blutgefäßen einschließlich der retinalen Gefäße ein Autoregulationssystem wirkt, welches auf einen erhöhten Kohlendioxid- bzw. Stickstoffoxidgehalt mit einer Gefäßdilatation und einem erhöhten Blutfluß reagiert, ist die Anreicherung mit Kohlendioxid/Stickstoffoxid medizinisch von besonderer Bedeutung. Beispielsweise stellt sich in Fluorcarbonen ein der Luftkonzentration entsprechender Partialdruck von Kohlendioxid ein. Dieser liegt aber im Gegensatz zu Sauerstoff unterhalb pysiologischer Werte. Während Kohlendioxid im arteriellen Blut Partialdrücke von 40mmHg und im venösen Blut von 45 mmHg besitzt, haben Fluorcarbone unter Raumluft einen Kohlendioxid-Partialdruck unter 1 mmHg.

Im Blut ist immer ein erhöhter Kohlendioxidgehalt mit einem tiefen Sauerstoffgehalt gekoppelt. Bei einem Behandlungsmittel kann auf einfache Weise dieses feststehende Verhältnis aufgehoben werden, indem erhöhte Kohlendioxidgehalte eingestellt werden, die zu einer Gefäßdilatation führen. Gleichzeitig können andererseits pysiologisch optimale Sauerstoffwerte eingestellt werden. Auch ein Stickstoffoxid enthaltendes Behandlungsmittel kann erst dann seine Wirkung entfalten, wenn der Sauerstoffpartialdruck so eingestellt wird, daß die Konkurrenzreaktion des freien Radikals NO mit Sauerstoff genügend unterdrückt wird.

Das erfindungsgemäße sauerstoffarme Fluorcarbon kann dann in an sich bekannter und in den eingangs genannten Druckschriften beschriebener Weise als Behandlungsmittel in der Ophtalmologie eingesetzt werden, beispielsweise als Glaskörperersatz bzw. Netzhauttamponade.

In einem Ausführungsbeispiel wurde zunächst durch Spülen mit Stickstoff der gesamte physikalisch gelöste Sauerstoff aus hochgereinigtem Perfluordecalin ausgetrieben. Dieser Vorgang wurde durch Sauerstoffmessungen überwacht. Ausgehend von einer Sauerstoffkonzentration von 8 Vol.-% wurde nach 30 Minuten ein Wert von 1 Vol.-% erreicht.

Dieses sauerstoffarme Perfluordecalin wurde anschließend steril filtriert und in jeweils ein Auge zweier Kaninchen injiziert wobei vor der Injektion eine an sich bekannte Gaskompression des Glaskörpers vorgenommen wurde. Nach der Gaskompression wurde das Gas gegen 1,2 ml des desoxygenierten Perfluordecalins intravitrenal ausgetauscht, so daß etwa zwei Drittel des Glaskörperraumes einschließlich des Bereiches der retinalen Blutgefäße mit Perfluordecalin gefüllt waren.

Nach Verweildauern zwischen zwei Tagen und sechs Wochen wurden die Augen auf Gefäßschädigungen untersucht. Hierbei ergab sich im Vergleich zu den unbehandelten Augen jeweils ein völlig normaler Befund.

Untersuchungen haben ergeben, daß sich die erfindungsgemäßen Stoffe auch als Behandlungsmittel in zur Ophtalmologie verwandten Bereichen eignen, insbesondere als Behandlungsmittel in der Gehirnchirurgie bei ischämiebedingten Störungen.

## Patentansprüche

1. Verwendung eines Fluorcarbons mit einem Gehalt an gelöstem Sauerstoff unter 6 Vol.-% zur Herstellung eines Behandlungsmittels für die Ophthalmologie.

2. Verwendung nach Anspruch 1,
**dadurch gekennzeichnet, dass** der Gehalt an gelöstem Sauerstoff unter 4 Vol.-% liegt.

3. Verwendung nach Anspruch 2,
**dadurch gekennzeichnet, dass** der Gehalt an gelöstem Sauerstoff unter 1 Vol.-% liegt.

4. Verwendung nach einem der vorhergehenden Ansprüche,
**dadurch gekennzeichnet, dass** das Behandlungsmittel ein intraokulares Behandlungsmittel ist.

5. Verwendung nach Anspruch 4,
**dadurch gekennzeichnet, dass** das Behandlungsmittel für die Netzhauttamponade und/oder zur Behandlung ischämischer Netzhauterkrankungen und/oder als Glaskörperersatzstoff verwendbar ist.

6. Verwendung nach einem der vorhergehenden Ansprüche,
**dadurch gekennzeichnet, dass** das Fluorcarbon ferner einen definierten Gehalt zwischen 0 und 20 Vol.-% eines weiteren Gases, insbesondere Kohlendioxid oder Stickstoffoxid aufweist.

## Claims

1. The use of a fluorocarbon having a content of dissolved oxygen below 6% by volume, for the manufacture of an opthalmological treatment agent.

2. The use according to claim 1,
**characterized in that** the dissolved oxygen content is less than 4% by volume.

3. The use according to claim 2,
**characterized in that** the dissolved oxygen content is less than 1% by volume.

4. The use according to any one of the previous claims,
**characterized in that** the treatment agent is an intraocular agent.

5. The use according to claim 4,
**characterized in that** the treatment agent can be used for retina tamponade and/or for the treatment of ischaemic retinal disorders and/or as a vitreous body replacement.

6. The use according to any of the previous claims,
**characterized in that** the fluorocarbon further comprises a defined amount from 0 to 20% by volume of a second gas, particularly carbon dioxide or nitrogen oxide.

## Revendications

1. Utilisation d'un fluorocarbure ayant une teneur en oxygène dissous inférieure à 6 % vol. pour fabriquer un agent de traitement pour usage ophtalmologique.

2. Utilisation selon la revendication 1, **caractérisée en ce que** la teneur en oxygène dissous est inférieure à 4 %vol.

3. Utilisation selon la revendication 2, **caractérisée en ce que** la teneur en oxygène dissous est inférieure à 1 % vol.

4. Utilisation selon l'une des revendications précédentes, **caractérisée en ce que** l'agent de traitement est un agent de traitement intraoculaire.

5. Utilisation selon la revendication 4, **caractérisée en ce que** l'agent de traitement peut être utilisé pour le tamponnement de la rétine et/ou le traitement de maladies ischémiques rétiniennes et/ou comme substitut du corps vitré.

6. Utilisation selon l'une des revendications précédentes, **caractérisée en ce que** le fluorocarbure a en outre une teneur définie en un autre gaz, en particulier le dioxyde de carbone ou l'oxyde d'azote, comprise entre 0 et 20 % vol.
